# EUROPEAN PATENT APPLICATION

(11) **EP 4 792 663 A2**
(43) Date of publication of application: **19.08.2026**
(21) Application number: 26169974.8
(22) Date of filing: 31.03.2020
(51) Int. Cl.: A23L 33/00

(54) **COMPOSITIONS AND METHODS FOR WEIGHT LOSS**

(30) Priority: 24.01.2020 US 202062965396 P
(62) Document number(s) of the earlier application(s) in accordance with Art. 76 EPC: 20916178.5 / 4 093 507
(71) Applicant: Barkey, Daniel, Q., Severance, CO 80550 (US)
(72) Inventor: Barkey, Daniel, Q., Severance, CO 80550 (US)
(74) Representative: Dehns Germany Partnerschaft mbB

(57) **Abstract**

The present disclosure provides a method to promote fat/weight loss of a subject, including the step of administering to the subject an effective amount of a composition comprising a lactate salt. The present disclosure also provides a method to prevent fat/weight gain of a subject while maintaining muscle mass.

## Description

### CROSS REFERENCE TO RELATED APPLICATION

This application claims priority to U.S. provisional application No. 62/965,396, filed January 24, 2020, which is herein incorporated by reference in its entirety.

### FIELD OF INVENTION

The disclosure relates to compositions and methods for promoting fat and/or weight loss. In particular, the disclosure relates to compositions and methods for promoting fat and/or weight loss while maintaining muscle mass.

### BACKGROUND

Many weight-loss strategies impose significant limits on calorie intake, as well as limitations on the amount of fat, and carbohydrates that can be consumed by an individual. However, due to the inherent causes of obesity, and overeating, dieting by itself is often unsuccessful in achieving individual goals.

First, high level of self-discipline is required by the dieter to lose significant amounts of weight. Second, significant risk of decreased muscle mass exists as a consequence of dieting. Because a functional TCA cycle is necessary to oxidize fat, when one is starved, the body begins to consume TCA cycle intermediaries in order to maintain the biosynthesis necessary for biological processes. This reduction in the number of TCA cycle intermediaries is called cataplerosis. The use of TCA cycle intermediaries for other biological processes diminishes the TCA cycle's capacity to oxidize acetyl CoA and therefore the ability of the body to generate energy during a catabolic state in which energy is desperately need. In a last-ditch effort to oxidize fat, the body breaks down muscle in order to liberate the carbon skeletons in gluconeogenic amino acids to increase the number of TCA cycle intermediaries (anaplerosis) which, in turn, increases the body's ability to turn fat into energy via oxidation through the TCA cycle. This loss of muscle comes at a very high biological cost because, at some point, the breakdown of muscle will have grave consequences on vital biological functions.

### SUMMARY

In one aspect, the present disclosure provides a method to promote weight loss or prevent weight gain of a subject, including administering to the subject an effective amount of a composition including a lactate salt.

In another aspect, the present disclosure provides a method to promote fat loss or prevent fat gain of a subject, including the step of administering to the subject an effective amount of a composition comprising a lactate salt.

In another aspect, the present disclosure provides a method to maintain muscle mass of a subject in a weight or fat loss process, including the step of administering to the subject an effective amount of a composition comprising a lactate salt, wherein the effective amount of lactate salt causes loss of fat in the subject while maintaining muscle mass of the subject.

In another aspect, the present disclosure provides a composition including a lactate salt and a carrier, wherein the lactate salt is present in the composition in an effective amount between 20 g and 100 g, and the lactate salt is at least one member selected from the group consisting of sodium lactate, potassium lactate, and calcium lactate, said composition being in an oral dosage form suitable for oral administration to a human.

### DETAILED DESCRIPTION

Provided are compositions and methods for promoting fat and/or weight loss, or preventing fat and/or weight gain.

### Definitions

For convenience, before further description of the present invention, certain terms used in the specification, examples and appended claims are collected here. These definitions should be read in light of the remainder of the disclosure and understood as by a person of skill in the art. Unless defined otherwise, all technical and scientific terms used herein have the same meaning as commonly understood by a person of ordinary skill in the art. The terms used throughout this specification are defined as follows, unless otherwise limited in specific instances.

The articles "a," "an" and "the" are used to refer to one or to more than one (i.e., to at least one) of the grammatical object of the article.

The term "about" herein is used to indicate that a value includes the inherent variation of error for the device, the method being employed to determine the value, or the variation that exists among the study subjects.

The terms "subject" as used herein refers to humans, non-human primates, and non-primate animals, including but not limited to, farm animals such as cattle, sheep, pigs, goats, and horses; domestic mammals such as dogs and cats; laboratory animals including rodents such as mice, rats, and guinea pigs; birds, including domestic, wild, and game birds such as chickens, turkeys, and other gallinaceous birds, ducks, geese, and the like. The term does not denote a particular gender.

The term "preventing weight gain" as used herein refers to controlling, arresting, and reducing weight gain. By preventing weight gain, for example, at least one or more of the following is achieved: decrease or maintenance in body fat or body weight, and/or cessation of weight gain.

The term "promoting weight loss" as used herein refers to achieving a weight reduction in the subject. For example, the administration of a composition as described herein may result in a weight reduction of, for example, at least 1, 2, 3, 5, 10, 15, 20, 25, 30, 35, 40, 45, or 50% of the body weight (as measured prior to the administration of the composition to the subject, i.e., baseline body weight). In the context of the present disclosure, weight reduction encompasses also the maintenance of a subject's weight.

The term "lactic acid" as used herein refers to 2-hydroxypropionic acid (aka 2-hydroxypropanoic acid) with the chemical formula C₃H₆O₃.The term "lactate salt" as used herein refers to a chemical compound in which the carboxylic proton of the "lactic acid" has been replaced by a pharmaceutically-acceptable cation. In some embodiments, the pharmaceutically-acceptable cation is a metal cation (such as sodium, potassium, calcium, or magnesium), an ammonium ion or a substituted ammonium, or phosphonium ion. The term "lactate" can refer to either stereoisomeric form of lactate (L-lactate or D-lactate), or a mixture thereof.

The term "composition" as used herein refers a system including a substance or more than one substance described herein. Compositions can be formulated, for example, for oral administration in unit dosage form (e.g., a tablet, capsule, caplet, gel cap, syrup, or liquid); for topical administration (e.g., as a cream, gel, lotion, or ointment); for intravenous administration (e.g., as a sterile solution or colloidal dispersion free of particulate emboli and in a solvent system suitable for intravenous use); or in any other formulation described herein.

The terms "administer" and "administering" as used herein refer to providing a composition to a subject in need. Multiple techniques of administering a composition exist in the art including, but not limited to, intravenous, oral, aerosol, parenteral, ophthalmic, pulmonary, and topical administration.

The term "effective amount" as used herein refers to an amount of a formulation, or composition to achieve a particular biological result. In certain embodiments, an effective amount treats or prevents a condition, e.g., ameliorates at least one sign or symptom associated with the condition.

The term "carrier" used herein refers to a material, composition or vehicle, such as a liquid or solid filler, diluent, excipient, solvent or encapsulating material, involved in carrying or transporting any supplement or composition, or component thereof, from one organ, or portion of the body, to another organ, or portion of the body. Each carrier must be "acceptable" in the sense of being compatible with the other ingredients of the formulation and not injurious to the subject. In one embodiment, the carrier is a "pharmaceutically acceptable carrier."

A "dosage form" includes any preparation, or combination of preparations, that provides a desired dosage. Hence a dosage form can include a single composition or a combination of several different compositions. A dosage form can "provide a daily dosage" in either a single unit dosage form or in multiple dosages taken at different times throughout a day. Hence a dosage form that includes multiple sub-dosage forms can provide the total daily dosage administered at different times during a day (for example at breakfast and lunch), and in different forms. In one embodiment, a dosage form is an artificial preparation that includes a pharmaceutical carrier.

An amount of lactate in a composition herein refers to an amount of formula (I) included in a composition. A percentage weight of lactate in a composition herein refers of a percentage weight of formula (I) in a composition.

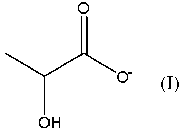

An amount of a lactate salt in a composition herein refers to an amount of formula (II) included in a composition. A percentage weight of a lactate salt in a composition herein refers to a percentage weight of formula (II) in a composition. 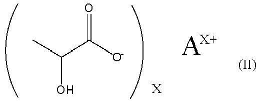 wherein X is 1, 2, 3, 4, or 5, A is a pharmaceutically-acceptable cation. In one embodiment, the pharmaceutically-acceptable cation is sodium, potassium, calcium, or magnesium. In another embodiment, the pharmaceutically-acceptable cation is an ammonium ion, or a substituted ammonium, or phosphonium ion. In one embodiment, the lactate salt is sodium lactate, potassium lactate, calcium lactate, or magnesium lactate.

### Compositions and Formulations

A composition of the present disclosure can be administered by a variety of methods known in the art. As will be appreciated by the skilled artisan, the route and/or mode of administration will vary depending upon the desired results. The compositions may be formulated for parenteral, oral, or local administration.

### Oral administration

Formulations for oral use include liquid or solid formulations containing a lactate salt in a mixture with non-toxic pharmaceutically acceptable excipients, and such formulations are known to the skilled artisan. These excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, and anti-adhesives (e.g., magnesium stearate, zinc stearate, stearic acid, silicas, hydrogenated vegetable oils, or talc). Other pharmaceutically acceptable excipients can be colorants, flavoring agents, plasticizers, humectants, buffering agents, and the like.

In one embodiment, the solid formulation is a tablet. The tablet may be uncoated or may be coated by known techniques, optionally to delay disintegration and absorption in the gastrointestinal tract and thereby providing a sustained action over a longer period. The coating may be adapted to release the compound in a predetermined pattern (e.g., in order to achieve a controlled release formulation) or it may be adapted not to release the agent(s) until after passage of the stomach (enteric coating). The coating may be a sugar coating, a film coating (e.g., based on hydroxypropyl methylcellulose, methylcellulose, methyl hydroxyethylcellulose, hydroxypropylcellulose, carboxymethylcellulose, acrylate copolymers, polyethylene glycols, and/or polyvinylpyrrolidone ), or an enteric coating (e.g., based on methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose). Furthermore, a time delay material such as, e.g., glyceryl monostearate or glyceryl distearate, may be employed. The solid tablet compositions may include a coating adapted to protect the composition from unwanted chemical changes (e.g., chemical degradation prior to the release of the active substances). The coating may be applied on the solid dosage form in a similar manner as that described in Encyclopedia of Pharmaceutical Technology.

The compositions of the present disclosure may be mixed together in a tablet, or may be partitioned. In one embodiment, the tablet may contain a first agent and a second agent, the first agent is contained on the inside of the tablet, and the second agent is on the outside, such that a substantial portion of the second agent is released prior to the release of the first agent. In one aspect, the first agent contains the composition disclosed herein.

Formulations for oral use may also be presented as chewable tablets, or as hard gelatin capsules wherein the lactate salt is mixed with an inert solid diluent (e.g., potato starch, lactose, microcrystalline cellulose, calcium carbonate, calcium phosphate, kaolin, and/or any pharmaceutically acceptable excipient or additive), or as soft gelatin capsules, wherein the active ingredient is mixed with water or an oil medium, for example, peanut oil, liquid paraffin, or olive oil. Formulations for oral use may also be presented as sachets.

Formulations for oral use may additionally be presented as extended release or prolonged release formulations/unit dosage forms. Powders and granulates may be prepared using the ingredients mentioned above under tablets and capsules in a conventional manner using, e.g., a mixer, a fluid bed apparatus, or spray drying equipment. The compounds of the present disclosure will be capable of extended shelf-life in such combinations.

Formulations for oral use may be a liquid formulation. In one embodiment, the liquid formulation is an aqueous liquid formulation.

In one embodiment, the composition includes between 1 wt% and 99 wt % a lactate salt, such as but not limited to, between 10 wt% and 90 wt %, between 20 wt% and 80 wt %, between 30 wt% and 70 wt %, between 40 wt% and 60 wt %, and between 45 wt% and 55 wt %.

### Dosage

The compositions of the present disclosure are formulated into pharmaceutically acceptable dosage forms by conventional methods known to those of skill in the art.

Actual dosage levels of the lactate salt in the compositions of the present disclosure may be varied so as to obtain an amount of the active ingredient (lactate) which is effective to achieve the desired response for a particular subject, composition, and mode of administration, without being toxic to the subject. The selected dosage level will depend upon a variety of pharmacokinetic factors including the activity of the particular compositions of the present disclosure employed, the route of administration, the time of administration, the rate of absorption of the particular agent being employed, the duration of the treatment, other substances, and/or materials used in combination with the particular compositions employed, the age, sex, weight, condition, general health and prior medical history of the subject being treated, and like factors well known in the medical arts. The dosage of any composition described herein may also depend on the amount or rate of weight loss desired, and the age, weight, and health of the subject to be treated.

One having ordinary skill in the art may readily determine and prescribe the effective amount of the composition required. For example, one may start doses of the substances of the present disclosure employed in the composition at levels lower than that required in order to
achieve the desired effects and gradually increase the dosage until the desired effect is achieved. In general, a suitable daily dose of a composition disclosed herein will be that amount of the substance which is the lowest dose effective to produce a desired effect. Such an effective dose will generally depend upon the factors described herein. The compositions of the present disclosure may conveniently be administered in unit dosage form and may be prepared by any of the methods well-known in the pharmaceutical art, for example, as described in "Remington: The Science and Practice of Pharmacy" (20th ed., ed. A. R. Gennaro, 2000, Lippincott Williams & Wilkins). In some embodiments, dosage levels are between about 2 g to about 200 g (e.g., 2, 4, 6, 8, 10, 20, 30, 40, 50, 60, 70, 80, 90, 100, 110, 120, 130, 140, 150, 160, 170, 180, 190, and 200 g) of lactate or a lactate salt per dose administered to a subject in need. In another embodiment, dosage levels are about 20 g to about 100 g (e.g., 20, 30, 40, 50, 60, 70, 80, 90, and 100 g) of the active ingredient lactate or a lactate salt.

In some embodiments, the composition may be administered to the subject in the dosage of between about 2 g to about 200 g of lactate or a lactate salt per day. In one embodiment, the composition may be administered to the subject lactate or a lactate salt in the dosage of about 2 g per day, 4 g per day, 6 g per day, 8 g per day, 10 g per day, 20 g per day, 30 g per day, 40 g per day, 50 g per day, 60 g per day, 70 g per day, 80 g per day, 90 g per day, 100 g per day, 110 g per day, 120 g per day, 130 g per day, 140 g per day, 150 g per day, 160 g per day, 170 g per day, 180 g per day, 190 g per day, or 200 g per day.

The composition may be administered to the subject in a single daily dose or in multiple doses per day. Administration may be one or multiple times daily, weekly (or at some other multiple day interval) or on an intermittent schedule, with that cycle repeated a given number of times (e.g., 2-10 cycles) or indefinitely. For example, a composition described herein may be administered once a day for, e.g., 2, 3, 4, 5, 6, 7, 8, 10, 15, 20, 25, 30 or more days. In another embodiment, a composition may be administered one or more times a day, e.g., 2, 3, 4, 5, 6, 7, 8, 9, 10, or more times a day. The composition may also be administered chronically, e.g., more than 30 days, e.g., 31 days, 40 days, 50 days, 60 days, 3 months, 6 months, 9 months, one year, two years, or three years). In one embodiment, compositions of the present disclosure will be administered for at least 30 days or more. In one embodiment, during the first week of treatment, the subject is administered a daily dose of about 2 g to 50 g active ingredient lactate or a lactate salt; if by the end of the week, the subject fails to report weight and/or fat loss, the daily dose may be increased to 60 to 80 g active ingredient lactate or a lactate salt. If by the end of the second week, the subject fails to report weight and/or fat loss, the daily dose may be increased to 90 to 100 active ingredient lactate or a lactate salt.

### Kits

The present disclosure provides a kit, which includes a composition including a lactate salt. In one embodiment, the kit may further include instructions to take the composition. In one embodiment, the instructions inform an individual to consume a certain amount of the composition per day. In one embodiment, the instructions inform an individual the time to consume the composition. In one embodiment, the instructions inform an individual to consume the composition at least 30, 40, 50, 60, 90, or 120 minutes before a physical exercise.

In another embodiment, the kit may further include instructions regarding a calorically restricted diet; and/or instructions to follow or not to follow any particular diet. In another embodiment, the kit may include instructions to follow or not to follow a prescribed exercise regimen.

In one aspect, the present disclosure provides a method to promote weight loss or prevent weight gain of a subject, including administering to the subject an effective amount of a composition including a lactate salt.

In another aspect, the present disclosure provides a method to promote fat loss of a subject, including the step of administering to the subject an effective amount of a composition comprising a lactate salt.

In another aspect, the present disclosure provides a method to maintain muscle mass of a subject in a weight or fat loss process, including the step of administering to the subject an effective amount of a composition comprising a lactate salt, wherein the effective amount of lactate salt causes loss of fat in said subject while maintaining muscle mass of said subject.

In one embodiment, the lactate salt is sodium lactate, potassium lactate, or calcium lactate. In another embodiment, the lactate salt is potassium lactate. In another embodiment, the lactate salt is a mixture of L-lactate and D-lactate. In another embodiment, the lactate salt is L-lactate.

In one embodiment, the effective amount of the composition comprises about 50-3,000 mg/kg of lactate/body weight of the subject. In another embodiment, the effective amount of the composition comprises about 100-2,000 mg/kg of lactate/body weight. In another embodiment, the effective amount of the composition comprises about 250-500 mg/kg of lactate/body weight. In another embodiment, the effective amount of the composition comprises about 400-500 mg/kg of lactate/body weight.

In another embodiment, the composition is administered orally. In another embodiment, the composition further includes a carrier, the carrier being selected from the group consisting of water, alcohol, capsules, powders, tinctures, liposomes, chewing gum, lozenges, candies, and food.

In another embodiment, the composition is in an oral dosage form. In another embodiment, the oral dosage form is selected from the group consisting of a chewable tablet, a quick dissolve tablet, an effervescent tablet, a hard gelatin capsule, a soft gelatin capsule, a powder, a reconstitutable powder, a suspension, an elixor, a caplet, a health bar, a liquid, a food and combinations thereof. In another embodiment, the oral dosage is selected from the group consisting of immediate release, extended release, pulsed release, delayed release, timed release, variable release, controlled release and combinations thereof.

In another embodiment, the oral dosage form includes 5-150 g of lactate. In another embodiment, the effective amount of the composition is 20-100 g of lactate per day. In another embodiment, the subject is participating in a hypocaloric dietary regimen. In another embodiment, the subject is participating in a physical exercise regimen. In another embodiment, the composition is administered more than one hour before exercise. In another embodiment, the composition is administered more than one and half hour or two hours before exercise.

In another aspect, the present disclosure provides a composition including a lactate salt and a carrier, wherein the lactate salt is present in the composition in an effective amount between 20 g and 100 g, and the lactate salt is at least one member selected from the group consisting of sodium lactate, potassium lactate, and calcium lactate, said composition being in an oral dosage form suitable for oral administration to a human.

Although the subject matter has been described in considerable detail with reference to certain embodiments thereof, other embodiments are possible. As such, the spirit and scope of the appended claims should not be limited to the description of the specific embodiments contained therein.

### EXAMPLE

The disclosure will now be illustrated with working examples, and which is intended to illustrate the working of disclosure and not intended to restrictively any limitations on the scope of the present disclosure. Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein.

### Example 1

Promoting Weight/Fat loss with a Composition Containing a Lactate Salt

This example discloses use of a composition containing a lactate salt in methods of promoting weight and/or fat loss of a subject while maintaining muscle mass. The lactate salt may be sodium lactate, potassium lactate, or calcium lactate. Dosage forms of the composition are administered to deliver desired daily dosages of lactate.

In the example, 5-150 g of lactate is administered to a subject to promote weight and/or fat loss of while maintaining muscle mass.

The present disclosure includes the following embodiments.

Paragraph 1A. A method to promote weight loss of a subject, comprising:
administering to the subject an effective amount of a composition comprising a lactate salt.

Paragraph 2A. A method to promote fat loss of a subject, comprising:
administering to the subject an effective amount of a composition comprising a lactate salt.

Paragraph 3A. A method to maintain muscle mass of a subject in a weight or fat loss process, comprising:
administering to the subject an effective amount of a composition comprising a lactate salt,
wherein said effective amount of lactate salt causes loss of fat in said subject while maintaining muscle mass of said subject.

Paragraph 4A. The method of any one of the preceding paragraphs, wherein the lactate salt is sodium lactate, potassium lactate, or calcium lactate.

Paragraph 5A. The method of any one of the preceding paragraphs, wherein the lactate salt is potassium lactate.

Paragraph 6A. The method of any one of the preceding paragraphs, wherein the lactate salt is a mixture of L-lactate and D-lactate.

Paragraph 7A. The method of any one of paragraphs 1A-5A, wherein the lactate salt is L-lactate.

Paragraph 8A. The method of any one of the preceding paragraphs, wherein the effective amount of the composition comprises about 50-3,000 mg/kg of lactate/body weight of the subject.

Paragraph 9A. The method of any one of the preceding paragraphs, wherein the effective amount of the composition comprises about 100-2,000 mg/kg of lactate/body weight.

Paragraph 10A. The method of any one of the preceding paragraphs, wherein the effective amount of the composition comprises about 250-500 mg/kg of lactate/body weight.

Paragraph 11A. The method of any one of the preceding paragraphs, wherein the effective amount of the composition comprises about 400-500 mg/kg of lactate/body weight.

Paragraph 12A. The method of any one of the preceding paragraphs, wherein the composition is administered orally.

Paragraph 13A. The method of any one of the preceding paragraphs, wherein the composition further comprises a carrier, the carrier being selected from the group consisting of water, alcohol, capsules, powders, tinctures, liposomes, chewing gum, lozenges, candies, and food.

Paragraph 14A. The method of any one of the preceding paragraphs, where the composition is in an oral dosage form.

Paragraph 15A. The method of paragraph 14A, wherein said oral dosage form is selected from the group consisting of a chewable tablet, a quick dissolve tablet, an effervescent tablet, a hard gelatin capsule, a soft gelatin capsule, a powder, a reconstitutable powder, a suspension, an elixor, a caplet, a health bar, a liquid, a food and combinations thereof.

Paragraph 16A. The method of paragraph 14A, wherein said oral dosage is selected from the group consisting of immediate release, extended release, pulsed release, delayed release, timed release, variable release, controlled release and combinations thereof.

Paragraph 17A. The method of any one of paragraphs 14A-16A, wherein the oral dosage form comprises 5-150 g of lactate.

Paragraph 18A. The method of any one of the preceding paragraphs, wherein the effective amount of the composition is 20-100 g of lactate per day.

Paragraph 19A. The method of any one of the preceding paragraphs, wherein the subject is participating in a hypocaloric dietary regimen.

Paragraph 20A. The method of any one of the preceding paragraphs, wherein the subject is participating in a physical exercise regimen.

Paragraph 21A. The method of paragraph 20A, wherein the composition is administered more than one hour before exercise.

Paragraph 22A. A composition comprising a lactate salt and a carrier, wherein said lactate salt is present in said composition in an effective amount between 20g and 100 g, and said lactate salt is at least one member selected from the group consisting of sodium lactate, potassium lactate, and calcium lactate, said composition being in an oral dosage form suitable for oral administration to a human.

## Claims

1. A method to promote weight loss in a subject, comprising:
administering to the subject an effective amount of a composition comprising a lactate salt, wherein the lactate salt is sodium lactate, or potassium lactate.

2. A method to promote fat loss in a subject, comprising:
administering to the subject an effective amount of a composition consisting of a lactate salt, wherein the lactate salt is sodium lactate, or potassium lactate.

3. A method to maintain muscle mass of a subject in a weight or fat loss process, comprising:
administering to the subject an effective amount of a composition comprising a lactate salt, wherein the lactate salt is sodium lactate, or potassium lactate,
wherein said effective amount of lactate salt causes loss of fat in said subject while maintaining muscle mass of said subject.

4. The method of claim 1, wherein the lactate salt is a mixture of L-lactate and D-lactate, or wherein the lactate salt is L-lactate.

5. The method of claim 1, wherein the effective amount of the composition comprises about 50-3,000 mg/kg of lactate/body weight of the subject, or wherein the effective amount of the composition comprises about 100-2,000 mg/kg of lactate/body weight, or wherein the effective amount of the composition comprises about 250-500 mg/kg of lactate/body weight, or wherein the effective amount of the composition comprises about 400-500 mg/kg of lactate/body weight.

6. The method of claim 1, wherein the composition further comprises a carrier, the carrier being selected from the group consisting of water, alcohol, capsules, powders, tinctures, liposomes, chewing gum, lozenges, candies, and food.

7. The method of claim 1, where the composition is in an oral dosage form.

8. The method of claim 7, wherein said oral dosage form is selected from the group consisting of a chewable tablet, a quick dissolve tablet, an effervescent tablet, a hard gelatin capsule, a soft gelatin capsule, a powder, a reconstitutable powder, a suspension, an elixor, a caplet, a health bar, a liquid, a food and combinations thereof.

9. The method of claim 7 or 8, wherein said oral dosage is selected from the group consisting of immediate release, extended release, pulsed release, delayed release, timed release, variable release, controlled release and combinations thereof.

10. The method of claim7, 8 or 9, wherein the oral dosage form comprises 5-150 g of lactate.

11. The method of claim 1, wherein the effective amount of the composition is 20-100 g of lactate per day.

12. The method of claim 1, wherein the subject is participating in a hypocaloric dietary regimen, or wherein the subject is participating in a physical exercise regimen.

13. The method of claim 12, wherein the subject is participating in a physical exercise regimen and the composition is administered more than one hour before exercise.

14. A composition comprising a lactate salt and a carrier, wherein said lactate salt is present in said composition in an effective amount between 20g and 100 g, and said lactate salt is at least one member selected from the group consisting of sodium lactate, and potassium lactate, said composition being in an oral dosage form suitable for oral administration to a human.
